# EUROPEAN PATENT SPECIFICATION

(11) **EP 4 076 286 B1**
(45) Date of publication and mention of the grant of the patent: **28.01.2026**
(21) Application number: 20842563.7
(22) Date of filing: 18.12.2020
(51) Int. Cl.: A61F 2/24

(54) **MULTI-LAYERED ELECTROSPUN HEART VALVE LEAFLETS**
MEHRSCHICHTIGE ELEKTROGESPONNENE HERZKLAPPENSEGEL
VALVES DE VALVULE CARDIAQUE ÉLECTROFILÉES MULTICOUCHES

(30) Priority: 20.12.2019 US 201962951530 P; 20.12.2019 US 201962951501 P
(43) Date of publication of application: 26.10.2022
(73) Proprietor: Xeltis AG, 8008 Zurich (CH)
(72) Inventor: GRAY, Yonatan, 5654 PD Eindhoven (NL); COX, Martijn Antonius Johannes, 6021 RC Budel (NL)
(74) Representative: FRKelly
(86) International application number: PCT/EP2020/086985
(87) International publication number: WO 2021/123137

(56) References cited:
- WO-A1-2016/130173
- WO-A1-2019/129636
- US-A1- 2002 090 725
- US-A1- 2015 088 247
- US-A1- 2016 317 295

## Description

### FIELD OF THE INVENTION

This invention relates to tissue engineering of heart valve leaflets.

### BACKGROUND OF THE INVENTION

Heart valves undergo tremendous stresses each heart beat while blood is being pumped through the cardiovascular system. Besides these stresses during each heartbeat, the heart valves also require significant durability lasting preferably a lifetime.

Prosthetic heart valves or engineered heart valves replace the natural heart valve in case of failure or injury to the natural heart valves. These prosthetic/engineered heart valves are single-layered heart valves and typically made from chemically fixated biological tissue (e.g. pericardium), yet they have limitations as they tend to heavily calcify long term and result in limited leaflet mobility and leaflet durability.

In some prosthetic/engineered heart valves materials are used to allow for Endogenous Tissue Restoration (ETR). The heart valve material would then be porous and bioabsorbable making the material capable for the heart valve to be absorbed by the body and replaced by natural tissue due to ingrowth of cells and nutrients into pores. However, the temporary nature of such an ETR-type heart valve may pose additional limitations to the durability of the heart valve.

The present invention addresses these problems of durability and mobility, and/or fraying of the leaflets, that originate from a single layered prosthetic/engineered heart valve and from heart valves that also desire ETR.

US 2015/088247 relates to a single-layer tissue sheet having a puncture strength of 2 kgf to 6 kgf. A valve, such as a heart valve, made of one or more leaflets formed from a single-layer tissue sheet. A method of making a tissue sheet having a puncture strength of 2 kgf to 5 kgf. The ultra-strong tissue sheets described therein have very long culture times, such as in excess of 20 weeks. Valves that comprise one or more leaflets made from the ultra-strong tissue sheets described therein may be delivered via trans-cathete aortic valve implantation.

WO 2019/129636 relates to a method of making a medical implant is provided by electrospinning a polymer solution to form a preform around a mandrel. The formed preform distinguishes an inner surface and an outer surface. The formed preform is removed from the mandrel and flipped inside-out resulting in the inner surface of the formed preform becoming the outer surface of the inside-out flipped preform, and the outer surface of the formed preform becoming the inner surface of the inside- out flipped preform. At least part of the inside-out flipped preform forms the medical implant such as e.g. an artificial heart valve, an artificial leaflet, an artificial graft, or an artificial vessel. The products made according to the method of this invention greatly improve the performance and durability of the medical implant.

US 2002/090725 relates to formation and use of electroprocessed collagen, including use as an extracellular matrix and, together with cells, its use in forming engineered tissue. The engineered tissue can include the synthetic manufacture of specific organs or tissues which may be implanted into a recipient. The electroprocessed collagen may also be combined with other molecules in order to deliver substances to the site of application or implantation of the electroprocessed collagen. The collagen or collagen/cell suspension is electrodeposited onto a substrate to form tissues and organs.

WO 2016/130173 relates to thin, biocompatible, high-strength, composite materials that are suitable for use in a prosthetic valve for regulating blood flow direction. In one aspect, the leaflet material maintains flexibility in high-cycle flexural applications, making it particularly applicable to high-flex implants such as a prosthetic heart valve leaflet. The leaflet material includes a coherent single layer and an elastomer, wherein the elastomer is present in the pores of the porous coherent single layer.

US 2016/317295 relates to systems and methods for producing biologic tissues. For example, this document provides electrospinning systems and culturing techniques to make biologic heart valve leaflets and fibrosa layers of native valve leaflet having nanofibrous substrate layer(s). In some implementations, a tri-layered leaflet with circumferentially, randomly, and radially oriented nanofibers that mimics morphologies of native leaflets

### SUMMARY OF THE INVENTION

The invention is defined in the appended claims. The disclosure provides in one embodiment, a medical implant engineered as one or more leaflets of a heart valve. The leaflet has two electrospun layers which are attached only at the edges of the two electrospun layers allowing for maximum and independent movability of the one layer relative to the other layer in the leaflet. The leaflet can be porous and bioabsorbable making the leaflet capable to be absorbed and replaced by natural tissue due to ingrowth of cells and nutrients into pores (ETR).

In another embodiment, the disclosure provides a medical implant engineered as a leaflet of a heart valve. In this case, the heart valve defines a base and a free edge. The leaflet has a single electrospun layer folded over itself to create and have a bi-layered design for the leaflet such that the free edge of the heart valve is the folded edge of the leaflet. Only the remaining un-folded edges of the electrospun layer are attached and incorporated to the base of the heart valve allowing for maximum and independent movability of the one layer relative to the other layer in the leaflet. Like the first embodiment, the leaflet can be porous and bioabsorbable making the leaflet capable to be absorbed and replaced by natural tissue due to ingrowth of cells and nutrients into pores (ETR).

The double layer design has a significantly improved mobility of the leaflets, without reduced durability, compared to an ordinary single-layer design. In addition, the folded double layer design also highly reduced the risk of fraying.

In yet another embodiment, the disclosure provides a medical implant engineered as a leaflet of a heart valve. In this case, a middle layer in between the two electrospun layers. The middle layer in one example could remain independently movable from the two electrospun layers, and in another example the middle layer could be adhered to the two electrospun layers.

In yet another embodiment, the disclosure provides a medical implant engineered as a leaflet of a heart valve. In this case, a middle layer is in between the bi-layered design. The middle layer in one example could remain independently movable from bi-layered design, and in another example the middle layer could be adhered to the bi-layered design.

The middle layer could be varied to be a: (i) porous and bioabsorbable making the middle layer capable to be absorbed and replaced by natural tissue due to ingrowth of cells and nutrients into pores, (ii) an electrospun layer which is porous and bioabsorbable making the middle layer capable to be absorbed and replaced by natural tissue due to ingrowth of cells and nutrients into pores, or (iii) not bioabsorbable and capable to be permeated with natural tissue due to ingrowth of cells and nutrients into pores.

The middle layer and the two electrospun layers are electrospun with the same material or different materials. In another embodiment, each of the two electrospun layers are electrospun with the same material or different materials. Similarly, the middle layer and the bi-layered design are electrospun with the same material or different materials.

The tri layer design also has a significantly improved mobility of the leaflets, without reduced durability, compared to an ordinary single-layer design. In addition, the folded double layer design for this tri layer design also highly reduced the risk of fraying.

In still another embodiment, the disclosure provides a medical implant engineered as a leaflet of a heart valve. In this case, the leaflet has two electrospun outer layers and a middle layer, whereby the middle layer is laminated between the two outer layers and not independently movable from the two electrospun outer layers. The middle layer could be varied to be a: (i) porous and bioabsorbable making the middle layer capable to be absorbed and replaced by natural tissue due to ingrowth of cells and nutrients into pores, (ii) an electrospun layer which is porous and bioabsorbable making the middle layer capable to be absorbed and replaced by natural tissue due to ingrowth of cells and nutrients into pores, or (iii) not bioabsorbable and capable to be permeated with natural tissue due to ingrowth of cells and nutrients into pores. The middle layer and the two electrospun layers are electrospun with the same material or different materials. Also, each of the two electrospun layers are electrospun with the same material or different materials.

### BRIEF DESCRIPTION OF THE DRAWINGS

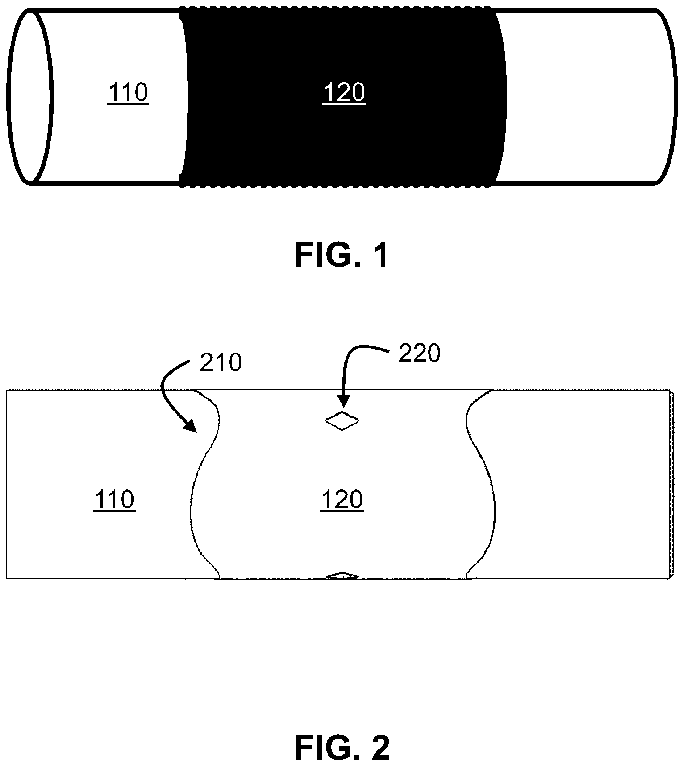

- **FIG. 1**: shows according to an exemplary embodiment of the disclosure electrospun fibers **120** deployed on a cylindrical metal mandrel **110.**

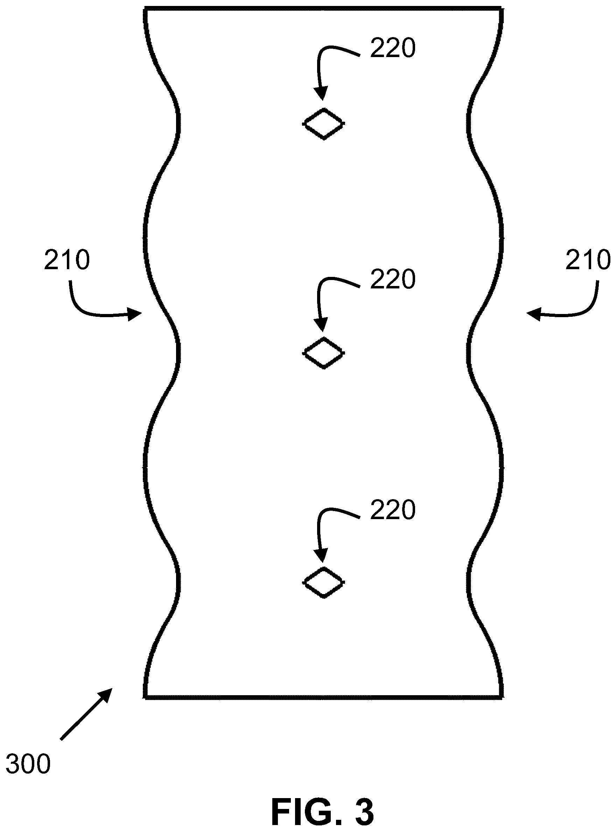

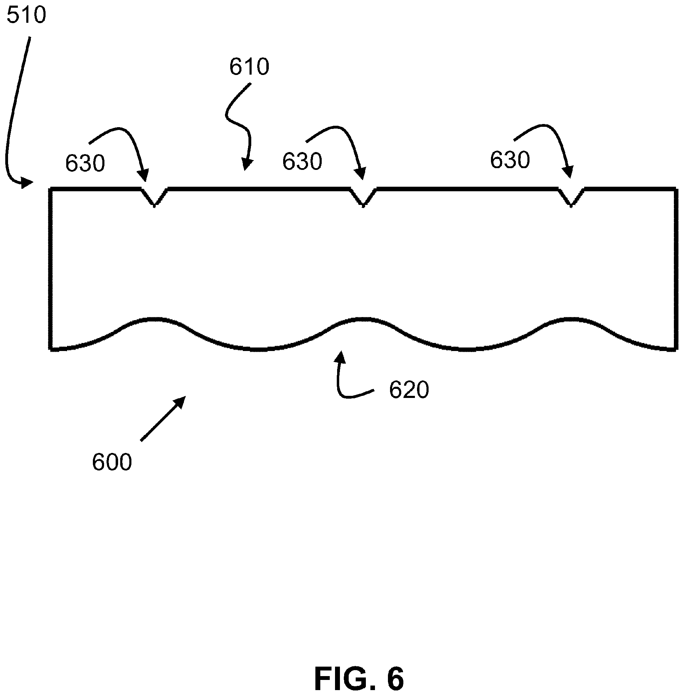

- **FIG. 2**: shows according to an exemplary embodiment of the disclosure the electrospun fibers **120** while still on the cylindrical metal mandrel **110** but now laser cut according to a laser-cut pattern as shown in **FIG. 3****.** Curved edges **210** are referred to as the heart valve base (see also **FIG. 6****),** and diamond shapes **220** are referred to as the shape commissures of the heart valve (see also **FIG. 6****).**
- **FIG. 3**: shows according to an exemplary embodiment of the disclosure a laser-cut pattern **300** to laser cut the electrospun fibers **120** while still on the cylindrical metal mandrel **110** (noted that this is a two-dimensional representation of a three-dimensional shape).

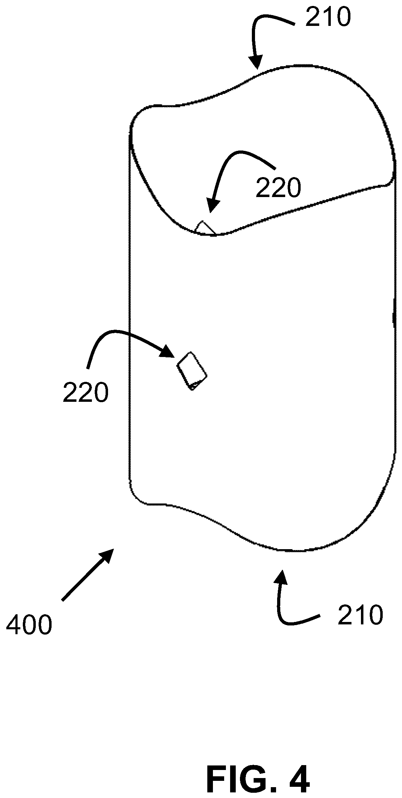

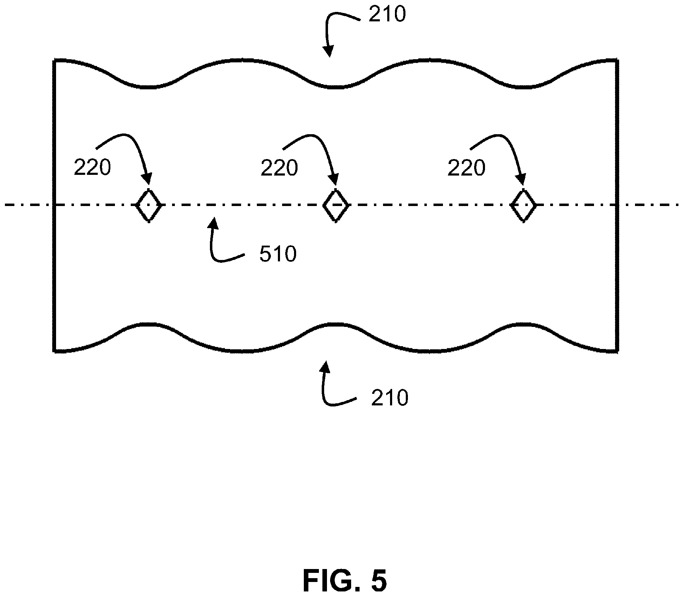

- **FIG. 4**: shows according to an exemplary embodiment of the disclosure the laser-cut electrospun heart valve shape **400** removed from the cylindrical metal mandrel (noted that the shape in **FIG. 4** is 90 degrees rotated relative to **FIGs. 1-2****).** This shape **400** is then to be folded across the diamond shapes **220** along the line **510** as shown in **FIG. 5****.**
- **FIG. 5**: shows according to an exemplary embodiment of the disclosure the fold line **510** where the shape **400** of **FIG. 4** is to be folded (noted that this is a two-dimensional representation of a three-dimensional shape). The fold-line becomes the free-edge **610** of the heart valve shape as shown in **FIG. 6** once folded.
- **FIG. 6**: shows according to an exemplary embodiment of the disclosure the folded heart valve shape **600** (noted that this is a two-dimensional representation of a three-dimensional shape). Curved edges **210** become the heart valve base **620** (see also **FIG. 2****),** and diamond shapes **220** become the V-shaped commissures **630** of the heart valve (see also **FIG. 2****).**

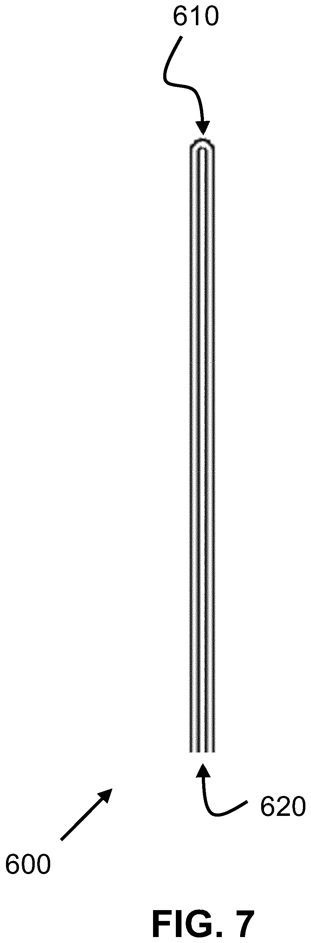

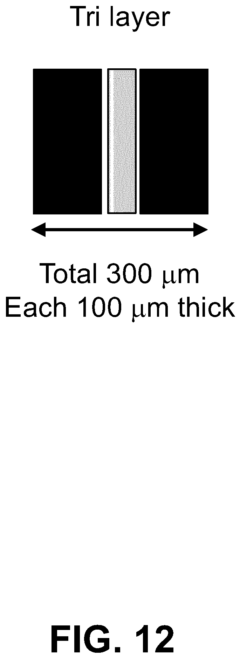

- **FIG. 7**: shows according to an exemplary embodiment of the disclosure a cross-sectional view of the folded heart valve shape **600** with folded free-edge **610** and heart valve base **620.** It is noted that this is two layers of the electrospun fibers folded at the fold-line **510 (****FIGs. 5-6****)** where the fold has become the free-edge **610.** In an alternate embodiment a heart valve leaflet can be engineered by having independent two electrospun layers which are then attached/ad only at the edges (similar as what is referred to as the free-folded-edge) of the two electrospun layers allowing for maximum and independent movability of the one layer relative to the other layer in the leaflet. Regarding the third design having a middle layer, the middle layer is placed in between the folded layers as a skilled artisan would readily appreciate (see also **FIG. 12****).**

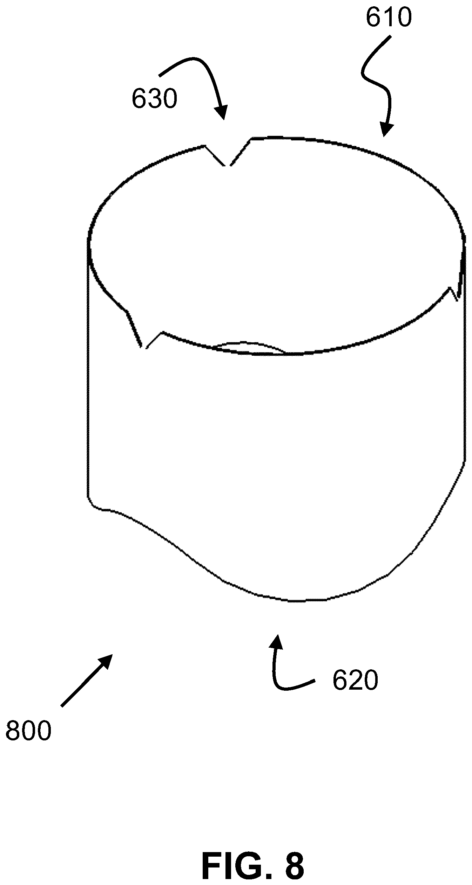

- **FIG. 8**: shows according to an exemplary embodiment of the disclosure a three- dimensional view of an electrospun folded heart valve **800** resulting from the steps as shown in **FIGs. 1-7** prior to suturing, gluing, stitching or generally adhering this heart valve to a heart valve frame.

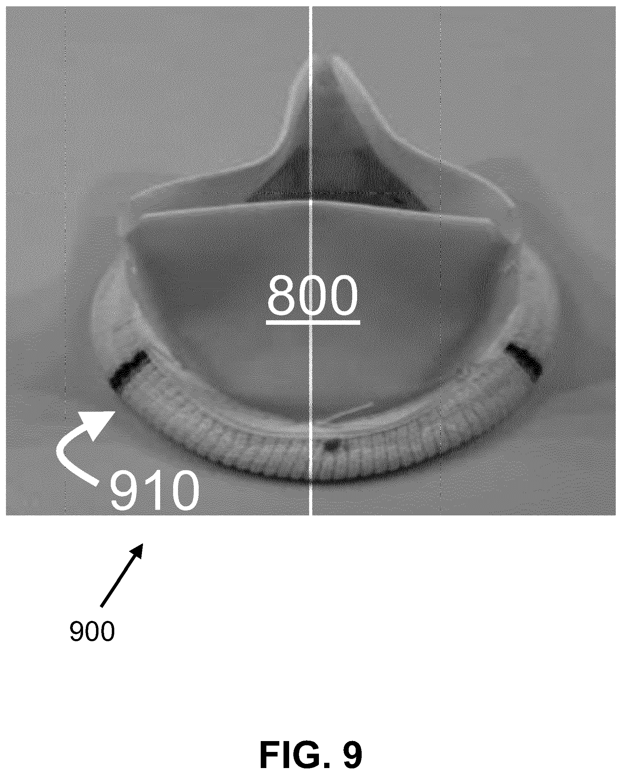

- **FIG. 9**: shows according to an exemplary embodiment of the disclosure an image of the assembly **900** of the electrospun folded heart valve, like 800, assembled to a heart valve base **910.**

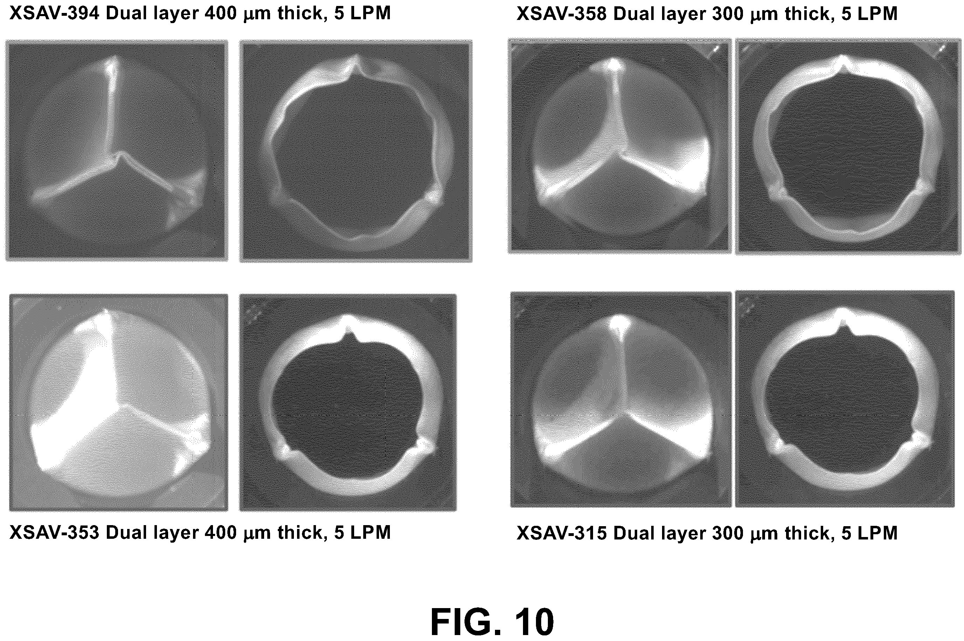

- **FIG. 10**: shows according to an exemplary embodiment of the disclosure fully closed and open phases of prototypes of heart valve assemblies produced by to the method steps provided herein and captured via a high-speed camera during hydrodynamic test.

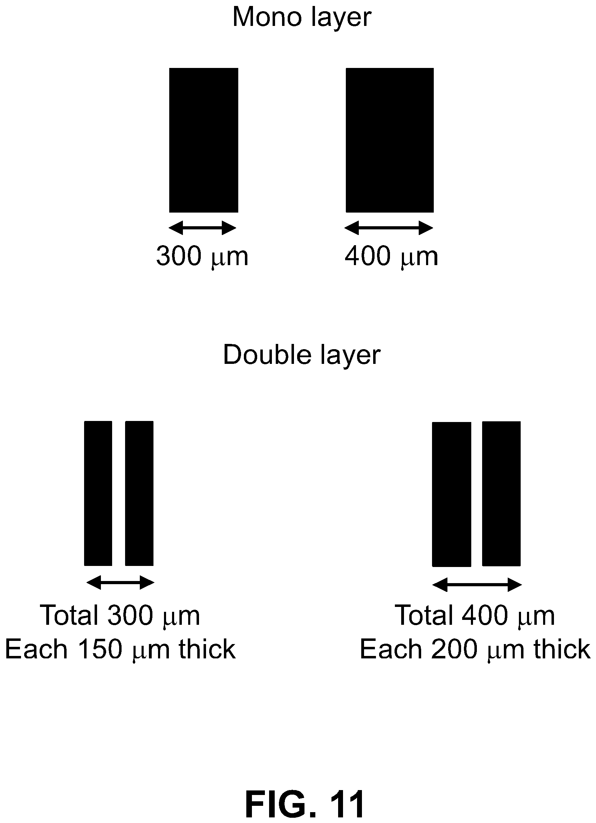

- **FIGs. 11-12**: show according to an exemplary embodiment of the disclosure the electrospun layer thicknesses and heart leaflet shapes that have been tested (see also **Tables 1-2).**

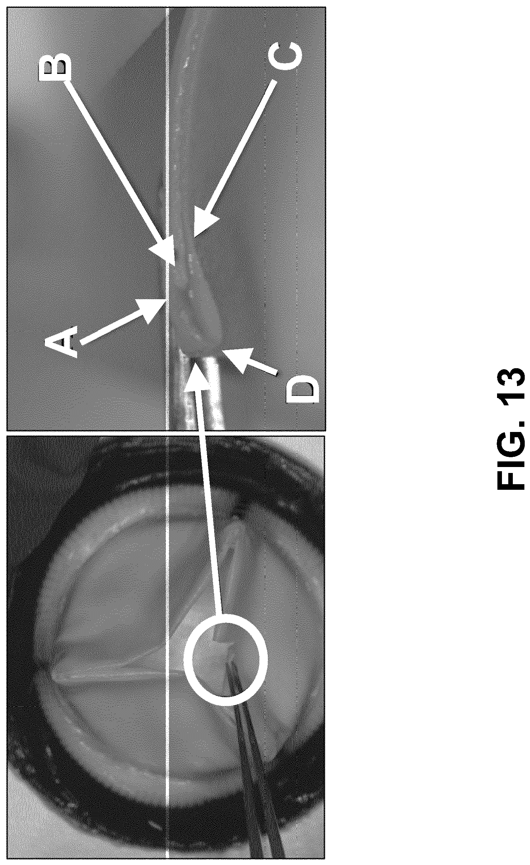

- **FIG. 13**: shows (left) an upper view on a tri-leaflet application in an aortic surgical valve, and (right) section view of a leaflet where (A) is the inner layer (a polymer as listed in the materials), (B) is the middle layer (PET woven sheet), (C) is the outer layer (a polymer as listed in the materials), and (D) is the folded free edge of the leaflet.

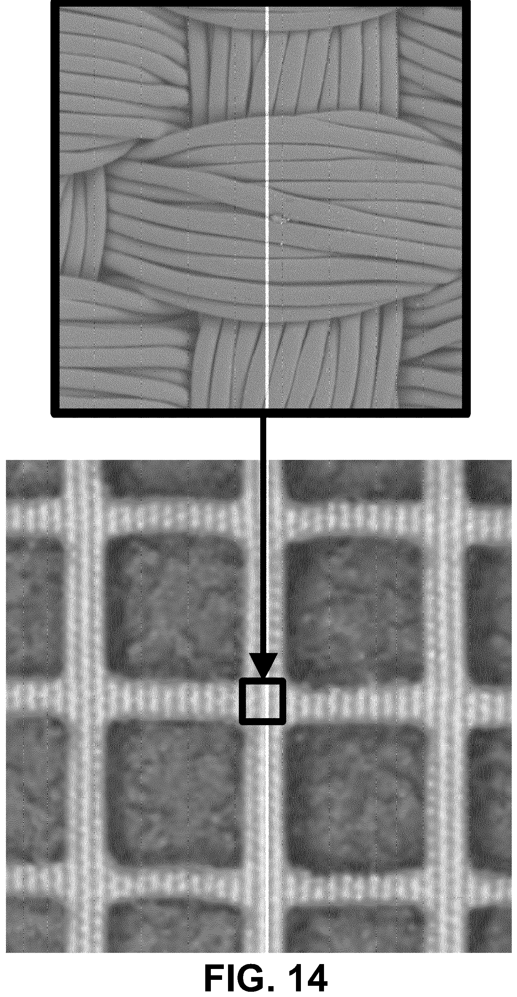

- **FIG. 14**: shows an example of the woven structure as an example of the middle layer. Bottom image shows a Polyethylene Terephthalate (PET) woven sheet laser cut into a mesh of square holes as an embodiment of an intermediate durable layer. The top image shows a magnification of the PET micro-structure via a scanning electrons microscopy (SEM) (Top). The PET durable mesh is used as a middle layer with dedicated holes to allow chronic remodeling between inner and outer XP polymer layers.

### DETAILED DESCRIPTION

Embodiments of the present disclosure advances the art and overcomes problems like durability, mobility, tearing and/or fraying by introducing a mutli-layered structure for the leaflets of the heart valve. A first design of a dual-layer is by having folded structure making a bi-layered structure, and a second design of a dual-layer is by adhering the edges of two independent electrospun layers at the free edge making a bi-layered structure. A third design is by having a dual-layered design with a layer in between the two layers of the dual-layered design.

In each design, the bi-layer leaflet of the heart valve is defined by two electrospun layers that preferably allow the maximum movement in between the layers to promote and ensure flexibility and mobility. In one embodiment, each layer in the bi-layer leaflet design could be produced from the same material, which is for example the materials as listed *infra.* In another embodiment, each layer in the bi-layer leaflet design could be produced from different materials. The latter is predominately true for the two independent electrospun layers that are adhered at the free edge.

The idea behind the bi-layer leaflet design is that two individual and independently moving layers in the design would be more flexible than a single layer leaflet if they would have the same overall thickness. The total thickness of the two layers combined in the bi-layer leaflet design is between 200 - 500 micrometers, and preferably 300 - 400 micrometers.

In the second design, it is preferred that the two layers are not adhered to each over the entire surface other allowing for the maximum movement independent from each other. To that effect, the two layers would be adhered or connected at the edges which comes to about 10-15% of the height of the heart valve leaflet. This could be accomplished by gluing, heat welding, or stitching the edges (or equivalent methods). As mentioned, in the first design, there is no need to adhere since that concept is accomplished by folding a sheet and only adhering the edges of the non-folder sides. The maximum movement ability between the two layers allows for more flexibility during bending, and this flexibility translates in better mobility and lower pressure gradients.

Directing to the first design, fibers are electrospun on a cylindrical metal mandrel **110** creating electrospun fibers **120 (****FIG. 1**). A list of useful fibers is provided towards the end of the description. Electrospun fibers **120** while still on the cylindrical metal mandrel **110** are then laser cut according to a laser-cut pattern **300 (****FIGs. 2-3****).** Curved edges **210** are referred to as the heart valve base (see also **FIG. 6****),** and diamond shapes **220** are referred to as the shape commissures of the heart valve (see also **FIG. 6****).** Once laser-cut, the electrospun heart valve shape **400** is removed from the cylindrical metal mandrel. Shape **400** is then folded across the diamond shapes **220** along the line **510 (****FIG. 5****).** In one example about three waves and about 3 diamonds are cut.

**FIG. 5** shows according to an exemplary embodiment of the disclosure the fold line **510** where the shape **400** of **FIG. 4** is to be folded (noted that this is a two-dimensional representation of a three-dimensional shape). Once folded, the fold-line becomes the free-edge **610** of the heart valve shape (**FIG. 6**). Once folded, curved edges **210** become the heart valve base **620** (see also

**FIG. 2****),** and diamond shapes **220** become the V-shaped commissures **630** of the heart valve (see also **FIG. 2****).** **FIG. 7** shows a cross-sectional view of the folded heart valve shape **600** with folded free-edge **610** and heart valve base **620.** It is noted that this is two layers of the electrospun fibers folded at the fold-line **510 (****FIGs. 5-6****)** where the fold has become the free-edge **610.** As mentioned, in the alternate and second design a heart valve leaflet can be engineered by having independent two electrospun layers which are then attached/ad only at the edges (similar as what is referred to as the free-folded-edge) of the two electrospun layers allowing for maximum and independent movability of the one layer relative to the other layer in the leaflet. **FIG. 8** shows the resulting electrospun folded heart valve **800** prior to suturing, gluing, stitching or generally adhering this heart valve to a heart valve frame. **FIG. 9** shows an image of the assembly **900** of the electrospun folded heart valve, like **800,** assembled to a heart valve base **910.**

Each of the layers of the bi-layer leaflet (first and second) design can be characterized as follows:
- Layer with micropores to allow Endogenous Tissue Regeneration (ETR). Pore size is about 1-100 micrometers.
- Electrospun layer with a fiber diameter of about 4-10 micrometers in one example, and 1-20 micrometers in another example.
- (Bio)absorbable layer, although not necessary the desirability depends on the application.
- Made from a polymer as listed *infra.*
- Thickness of the layer would typically be between 80-200 micrometers in one example, and 50-300 micrometers in another example.
- The total thickness of the two layers combined in the bi-layer leaflet design is between 200 - 500 micrometers, and preferably 300 - 400 micrometers.

In the third design, a middle layer is placed in between the two layers of the dual-layered design. This means a middle layer added to the first design and a middle layer added to the second design as discussed above whereby the first design is by having folded structure making a bi-layered structure, and the second design by adhering the edges of two independent electrospun layers at the free edge making a bi-layered structure. In one option, the middle layer could be assembled in between such that only the remaining un-folded edges of the electrospun layer and the middle layer are attached allowing for maximum and independent movability of the layers relative to each other. In another option, the middle layer could maintain maximum and independent movability without any attachment.

In one embodiment, the tri-layer leaflet design could be produced from the same material, which is for example the materials as listed *infra.* In another embodiment the tri-layer leaflet design could be produced from different materials. The key aspect of the middle layer relative to the outer layers is that the middle layer is of such material characteristics that they would increase the heart valve durability yet maintaining the mobility of the overall leaflets and therewith heart valve. In general, the middle layer is preferably more durable to improve the overall durability of the device even further. This could be achieved by choosing a different material or by a different texture (e.g. solid / woven / electrospun sheet with same or less porosity than the outer layers, see e.g. **FIG. 14****).**

In general, the middle layer could be produced from a biodegradable or non-biodegradable material. Preferably it is produced in a way that degradation time is reduced compared to the outer layers. The middle layer could be ETR compatible or non-ETR compatible (then being incorporated into the created tissue). Preferably it will be ETR compatible when a biodegradable set-up is chosen. Possibly (ideally) the middle layer has micropores and/or micropores to allow ETR through its pores and/or to allow the outer layers to attach to each other through the electrospinning (the latter requires macropores and a relatively thin middle layer).

In an alternate embodiment, the middle layer could be produced by electrospinning. Electrospinning parameters should be adapted to create a higher strength (via variation of the fiber alignment, pore size, material, fiber thickness, etc.). In particular the fibers have a preferred orientation along the circumference of the leaflets similar to native leaflet orientation. Alternatively, the middle layer could be a film or a 3D printed structure.

In one embodiment, the material of the middle layer is biocompatible, durable and should either degrade very slow or even stay in the body. In one example, a thin woven fabric made of PET could be used. This layer has a different texture than electrospun outer layers where a tight bundle of filaments is constantly woven to increase strength. Permeability of the middle layer could be introduced by creating a patterned perforation of the support structure. The PET woven structure should be very durable to dynamic shear stress in comparison to the outer layer and its thickness is set to 60-100 microns. Architecture of woven structure (e.g. - number of filaments per bundle, yarn angle, permeability, patterned perforation, suture retention strength, etc.) can be tuned and tailored to a specific leaflet design (i.e. - to conform to a specific valve frame) (see e.g. **FIG. 14****).**

In a second example a thin film 60-100 microns thick-casted of polymer solution (e.g. as described in the list *infra*) (not electrospun) could be used for a slow pace degradable middle layer with high durability potential. This middle layer will stabilize the leaflet while the ETR process takes place even later throughout the whole lifetime of the artificial valve in the body. Experiments have been performed and this tri-layer design shows an improved durability compared to simple double- or mono-layer designs. The middle layer would thereby absorb the stresses. Permeability of the middle layer could be introduced by creating a patterned perforation of the support structure.

In another example, the PET structure contains macro and/or micropores to allow ETR and/or a connection between the outer layers through electrospinning.

There are several methods to produce such multi-layer membranes or leaflets. According to one embodiment the outer layers are produced (e.g. by electrospinning). Then these layers are connected around the middle layer by heat welding, gluing or other methods.

The middle layer could also be described as a heart leaflet support structure embedded in between electrospun layers. The heart leaflet support structure could, without limitation:
- Be sufficiently porous to allow ETR;
- Sufficiently "open" to allow the inner and outer electrospun layer to laminate to each other.
- May or may not be a non-degradable layer;
- Be a woven (PET) mesh, suture wires, (metal e.g. nitinol) braided/knitted mesh; and/or
- Potentially post-processed to generate local porosity/open cell structure.

Further, the middle layer could also be described as a heart leaflet support structure embedded in between electrospun layers. The heart leaflet support structure could, without limitation:
- Be sufficiently porous to allow ETR;
- Made of a film (same material or material class as electrospun outer layers);
- Laminated to the electrospun layers, but it does not necessarily have to be sufficiently open to allow the electrospun layers to laminate to each other (i.e. they stick to the film already);

In the case where the free edge of the layers is adhered one could envision this to be about 10-15% of the height of the leaflet, which is about 1 mm. The rationale is to keep the free edge from delaminating and therefore to account for the about 10-15% of height which is (fully) laminated. The middle layer at its top will not reach the free edge, but about 1mm below it. Yet in still another embodiment, for the dual-layer designs or the tri-layer design with the middle (heart leaflet support structure) lamination could be desired in certain applications versus allowing the layers to move freely with respect to each other.

### Testing

To assess and quantify in-vitro mobility of a tested valve, a commercially available pulse duplicator (A "Hydrodynamic tester" by BDC Labs, USA) was used. In this classical test apparatus, a valve is placed in a fluidized chamber with flow and pressure control. Compressed air combined with test fluid is utilized to mimic physiological conditions and blood vessels compliance. The hydrodynamic tester interface analyzes valve life performance, essential parameters are measured and quantified in an open phase of the valve (i.e. effective orifice area (EOA) [cm²], max and mean positive pressure difference (PPD) [mmHg]) and in a closed phase of the valve (i.e. closing volume (CV) [%], regurgitation fraction (RF) [%]).

The tester can accommodate various heart rates and cardiac outputs under desired pressures replicating range of physiological conditions of cardiovascular system.

To assess and quantify the improved durability of a multi-layer valve, a classic commercially available valve durability tester was used (An "accelerated wear tester" by BDC Labs, USA). In such a tester, the valve is placed in a pressure-controlled, sealed chamber while being submitted to fluid flow pushed through its leaflets via a linear motor and a piston. Piston location controls the opening and closing of the leaflets and frequency of the motor dictates the speed of a valve cycle (Hz). Dynamic pressure gradient over a closed valve is set to simulate physiological conditions. A valve tested can usually be run in frequency of 5-25 Hz, where for this application in consideration with a polymer and its viscoelastic properties, a frequency of 10 Hz was chosen. A cycle is counted only if 5% or more of the cycle duration while the valve is closed, is endured under a specific pressure gradient (i.e. - target pressure). In this application, target pressure was set at 100mmHg.

Additional parameters are measured to assess valve mobility using a high-speed camera from various views. These parameters are not always standardized (according to ISO-5840), but could be valuable for characterization of valve dynamic performance, such as: leaflet to leaflet synchronization and overall symmetry in opening and closing phases, closed leaflet sagging, commissural deflection, etc. Some of these parameters are addressed mainly in product design development.

In **FIG. 10** and **Table 1** show results of in-vitro testing for mobility of bi-layer and single-layer valves of various leaflet thicknesses as shown in **FIG. 11****.** **FIG. 10** shows fully closed and open phases of prototypes of heart valve assemblies and captured via a high-speed camera during hydrodynamic test. The experiments show that a double layer design has a significantly improved mobility of the leaflets, without reduced durability, compared to an ordinary single-layer design. In addition, the folded double layer design also highly reduced the risk of fraying.

**Table 1: Single-layer vs. Bi-layer leaflets in hydrodynamic tester. First comparison shows that the embodiment of a 400µm thick Bi-layer leaflets (n=2) is as mobile as Single-layer leaflets, while the valve is in opening phase (effective orifice area and pressure gradient are relatively the same) and inferior in closing phase (larger closing volume leads to larger regurgitation fraction). Second comparison suggests that the embodiment of a 300µm thick Bi-layer leaflet (n=1) is more mobile than a Single-layer leaflet in opening phase (higher effective orifice area, lower pressure gradient), as mobile as a Single-layer leaflet in closing phase (similar closing volume).**

| **Normotensive aortic conditions (CO= 5 LPM, P=120/80 mmHg)** | | | | |
|---|---|---|---|---|
| **Valve** | **EOA [cm²]** | **PPD [mmHg]** | **CV [%]** | **RF [%]** |
| **XSAV-394, bi-layer, 400µm total thickness** | **2.7** | **5.8** | **10.2** | **10.8** |
| **XSAV-395, bi-layer, 400µm total thickness** | **3.0** | **4.6** | **11.9** | **12.8** |
| **XSAV-353, single-layer, 400µm total thickness** | **2.6** | **6.3** | **5.8** | **5.9** |
| **XSAV-355, single-layer, 400µm total thickness** | **3.0** | **4.6** | **6.3** | **6.5** |
| **XSAV-358, bi-layer, 300µm total thickness** | **3.2** | **3.8** | **7.4** | **7.9** |
| **XSAV-315, single-layer, 300µm total thickness** | **2.5** | **6.6** | **7.1** | **11.5** |

**Table 2: Results of a durability comparison between a tri-layer prototype and three mono-layer prototypes showing an improvement of at least one magnitude in cycles completed up to failure of the leaflets where required pressure gradient was unable to be reached. In conclusion, the tri-layer prototype had reached and passed the ISO standard for heart valve durability of 400M cycles. This shows the potential to pursue ETR in a heart valve with competitive durability compared to the state of the art (biological tissue leaflets).**

| **#** | **Type** | **Durability cycle count [Million]** | **Failure mode** |
|---|---|---|---|
| **1** | **Tri-layer, 300µm total thickness, welded posts** | **405** | **Commissural tear** |
| **2** | **Mono-layer 400µm thick, welded posts** | **10 (failed)** | **Commissural tear** |
| **3** | **Mono-layer 400µm thick, welded posts** | **27 (failed)** | **Commissural tear** |
| **4** | **Mono-layer 400µm thick, welded posts** | **34 (failed)** | **Commissural tear** |

### Materials

The electrospun material referenced in herein may comprise the ureido-pyrimidinone (UPy) quadruple hydrogen-bonding motif (pioneered by Sijbesma (1997), Science 278, 1601- 1604) and a polymer backbone, for example selected from the group of biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

The same result may be obtained with alternative, non-supramolecular polymers, if properties are carefully selected and material processed to ensure required surface characteristics. These polymers may comprise biodegradable or non-biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

## Claims

1. A medical implant, comprising:
a leaflet of an engineered heart valve, wherein the leaflet has two electrospun layers which are attached only at a free edge and a base of the heart valve, wherein attachment of the two layers at the free edge of the heart valve is about 10-15% of the height of the leaflet, and wherein the total thickness of the two layers combined is between 200 and 500 micrometers, allowing independent movability of the one layer relative to the other layer in the leaflet.

2. The medical implant as set forth in claim 1, wherein the leaflet is porous and bioabsorbable making the leaflet capable to be absorbed and replaced by natural tissue due to ingrowth of cells and nutrients into pores.

3. The medical implant as set forth in claim 1, further comprising a middle layer in between the two electrospun layers, wherein the middle layer remains independently movable from the two electrospun layers.

4. The medical implant as set forth in claim 3, wherein the middle layer is (i) porous and bioabsorbable making the middle layer capable to be absorbed and replaced by natural tissue due to ingrowth of cells and nutrients into pores, (ii) an electrospun layer which porous and bioabsorbable making the middle layer capable to be absorbed and replaced by natural tissue due to ingrowth of cells and nutrients into pores, or (iii) not bioabsorbable and capable to be permeated with natural tissue due to ingrowth of cells and nutrients into pores.

5. The medical implant as set forth in claim 3, wherein the middle layer and the two electrospun layers are electrospun with the same material or different materials.

6. The medical implant as set forth in claim 3, wherein the each of the two electrospun layers are electrospun with the same material or different materials.

## Patentansprüche

1. Medizinisches Implantat, umfassend:
ein Segel einer konstruierten Herzklappe, wobei das Segel zwei elektrogesponnene Schichten aufweist, die nur an einem freien Rand und einer Basis der Herzklappe befestigt sind, wobei die Befestigung der zwei Schichten am freien Rand der Herzklappe etwa 10-15 % der Höhe des Segels beträgt und wobei die Gesamtdicke der zwei Schichten zusammen zwischen 200 und 500 Mikrometer beträgt, was eine unabhängige Beweglichkeit der einen Schicht relativ zu der anderen Schicht in dem Segel ermöglicht.

2. Medizinisches Implantat nach Anspruch 1, wobei das Segel porös und bioresorbierbar ist, wodurch das Segel geeignet ist, aufgrund des Einwachsens von Zellen und Nährstoffen in Poren durch natürliches Gewebe absorbiert und ersetzt zu werden.

3. Medizinisches Implantat nach Anspruch 1, ferner umfassend eine mittlere Schicht zwischen den zwei elektrogesponnenen Schichten, wobei die mittlere Schicht unabhängig von den zwei elektrogesponnenen Schichten beweglich bleibt.

4. Medizinisches Implantat nach Anspruch 3, wobei die mittlere Schicht (i) porös und bioresorbierbar ist, wodurch die mittlere Schicht geeignet ist, aufgrund des Einwachsens von Zellen und Nährstoffen in Poren durch natürliches Gewebe absorbiert und ersetzt zu werden, (ii) eine elektrogesponnene Schicht ist, die porös und bioresorbierbar ist, wodurch die mittlere Schicht geeignet ist, aufgrund des Einwachsens von Zellen und Nährstoffen in Poren durch natürliches Gewebe absorbiert und ersetzt zu werden, oder (iii) nicht bioresorbierbar ist und geeignet ist, aufgrund des Einwachsens von Zellen und Nährstoffen in Poren von natürlichem Gewebe durchdrungen zu werden.

5. Medizinisches Implantat nach Anspruch 3, wobei die mittlere Schicht und die zwei elektrogesponnenen Schichten aus dem gleichen Material oder unterschiedlichen Materialien elektrogesponnen sind.

6. Medizinisches Implantat nach Anspruch 3, wobei jede der zwei elektrogesponnenen Schichten aus dem gleichen Material oder unterschiedlichen Materialien elektrogesponnen ist.

## Revendications

1. Implant médical, comprenant :
une valve de valvule cardiaque artificielle, dans lequel la valve présente deux couches électrofilées qui sont fixées uniquement à un bord libre et à la base de la valve cardiaque, dans lequel la fixation des deux couches au bord libre de la valve cardiaque est d'environ 10 à 15 % de la hauteur de la valve, et dans lequel l'épaisseur totale des deux couches combinées étant comprise entre 200 et 500 micromètres, permettant une mobilité indépendante de chaque couche par rapport à l'autre dans la valve.

2. Implant médical selon la revendication 1, dans lequel la valve est poreuse et bioabsorbable, ce qui permet à la valve d'être absorbée et remplacée par du tissu naturel grâce à la croissance de cellules et de nutriments dans les pores.

3. Implant médical selon la revendication 1, comprenant également une couche intermédiaire entre les deux couches électrofilées, dans lequel la couche intermédiaire reste mobile indépendamment des deux couches électrofilées.

4. Implant médical selon la revendication 3, dans lequel la couche intermédiaire est (i) poreuse et bioabsorbable, ce qui la rend capable d'être absorbée et remplacée par du tissu naturel grâce à la croissance de cellules et de nutriments dans les pores, (ii) une couche électrofilée poreuse et bioabsorbable, ce qui la rend capable d'être absorbée et remplacée par du tissu naturel grâce à la croissance de cellules et de nutriments dans les pores, ou (iii) non bioabsorbable et capable d'être imprégnée par du tissu naturel grâce à la croissance de cellules et de nutriments dans les pores.

5. Implant médical selon la revendication 3, dans lequel la couche intermédiaire et les deux couches électrofilées sont électrofilées avec le même matériau ou des matériaux différents.

6. Implant médical selon la revendication 3, dans lequel chacune des deux couches électrofilées est électrofilée avec le même matériau ou avec des matériaux différents.
